# EUROPEAN PATENT SPECIFICATION

(11) **EP 0 639 968 B1**
(45) Date of publication and mention of the grant of the patent: **13.03.1996**
(21) Application number: 93910793.4
(22) Date of filing: 26.04.1993
(51) Int. Cl.: A61K 7/32, A61K 7/48

(54) **ANTIPERSPIRANT GEL STICK COMPOSITION**
SCHWIESSHEMMENDE GELSTIFTZUSAMMENSETZUNG
COMPOSITION DE GEL ANTISUDORAL EN BATON

(30) Priority: 12.05.1992 US 881934
(43) Date of publication of application: 01.03.1995
(73) Proprietor: THE PROCTER & GAMBLE COMPANY, Cincinnati, Ohio 45202 (US)
(72) Inventor: HOFRICHTER, Brian, David, Cincinnati, OH 45211 (US); GARDLIK, John, Michael, Cincinnati, OH 45231 (US)
(74) Representative: Brooks, Maxim Courtney
(86) International application number: PCT/US93/03887
(87) International publication number: WO 93/23008

(56) References cited:
- DE-A- 2 260 640
- GB-A- 1 485 694
- PATENT ABSTRACTS OF JAPAN vol. 016, no. 544 (C-1004)13 November 1992
- CHEMICAL ABSTRACTS, vol. 85, no. 2, 12 July 1976, Columbus, Ohio, US; abstract no. 8510310, HONMA, MASAO ET AL. 'Gel-like cosmetic preparations containing N- acylamino acid derivatives' page 318 ; See abstract
- PATENT ABSTRACTS OF JAPAN & JP-A-61206450 (TAKASAGO PERFUMERY KK; AJINOMOTO KK) 12 September 1986
- PATENT ABSTRACTS OF JAPAN vol. 013, no. 199 (C-594)11 May 1989
- PATENT ABSTRACTS OF JAPAN vol. 014, no. 456 (C-0765)2 October 1990
- PATENT ABSTRACTS OF JAPAN vol. 13, no. 515 (C-655)(3863) 17 November 1989
- PATENT ABSTRACTS OF JAPAN vol. 012, no. 148 (C-493)7 May 1988

## Description

### FIELD OF THE INVENTION

The present invention are antiperspirant gel stick compositions to prevent perspiration and body odors having superior stability, low residue on the skin, low skin irritation, and excellent aesthetic characteristics. Furthermore, these compositions can be easily prepared by conventional techniques.

### BACKGROUND OF THE INVENTION

Personal hygienic habits typically include a means for reducing of human body odors. These habits include routine bathing or washing the body, particularly the axilla, and treating said areas with a compositions to retard odor formation. Among such compositions are deodorants and antiperspirants.

Deodorants are generally regarded as odor masking compositions which inhibit odors by retarding the growth of bacteria. Absent bacteria, the sweat produced by the body is not subjected to breakdown to odoriferous fatty acids. Antiperspirants also prevent formation of odoriferous fatty acids by retarding the growth of bacteria. However, unlike deodorants, antiperspirants prevent the formation of sweat which could be attacked by bacteria.

Antiperspirants are well known in the art, and typically comprise an astringent material in a suitable carrier. Astringent materials typically used in antiperspirants are metal salts, particularly aluminum and zinc metal complexes. Said metal salts is disclosed in Plechner, Antiperspirants and Deodorants, 2 Cosmetics, Science and Technology, Balsam and Sagarin, 374-400, 1972.

In addition to these above disclosure, Plechner also discloses that antiperspirants can take a number of different forms, each dependent on the ingredients used in addition to the above mentioned astringent metallic salts. The forms disclosed therein include liquids, solids, and semi-solids. Examples of these forms include lotions, solid sticks, and creams; the most popular being solid sticks.

Antiperspirant sticks are well known in the art. For example antiperspirant sticks are disclosed in U.S Patent 2,890,987, Hilfer, issued June 16, 1959; and U.S. Patent 3,255,082, Barton et al, issued June 7, 1966. There are three main types of such stick formulations. These are compressed powder sticks, gel sticks , and wax sticks. While each may have advantages in certain use situations, each also has disadvantages.

Antiperspirant compressed powder sticks mainly comprise powdery materials such as talc, powdered antiperspirant actives, and a binder which allows the stick to be formed using compressive forces. Although these sticks are generally stable, application to the skin is difficult since they are brittle and hard. Furthermore, since they are white, and opaque, they leave a cosmetically undesirable dusty residue after application.

Wax sticks are disclosed in U.S. Patents 4,822,603, Farris et al, issued April 18, 1989; 4,919,934, Deckner, issued April 24, 1990; and 4,944,937, McCall, issued July 31,1990. Wax sticks comprise liquid materials generally selected based on the desired physical properties they impart to the stick. Generally these liquid materials include silicones, hydrocarbon oils, esters, propoxylated and/or ethoxylated alcohols, and mixtures thereof. In addition to the liquid material, solidifying agents are added to impart stable physical structure to the stick. Typical solidifying agents used in wax sticks include fatty alcohols, fatty acids, fatty esters, triglycerides, and waxes having melting points from about 65°C to about 110°C. These waxes and fatty materials, however, can promote cosmetically unacceptable products due to such factors such as hardness, greasiness, stickiness, and difficult application. Furthermore, the crystalline nature of these wax formulations contributes to opacity and produces an undesirable residue during use.

Gel sticks are also well known in the art. These sticks contain a liquid material and solidifying agents. The liquid materials typically are water, lower monohydric alcohols, polyhydric alcohols, and mixtures thereof. The solidifying agents most often used include fatty acid soaps, and dibenzylidine monosorbitol acetals (hereinafter DBS). See the following patents for soap gels: U.S. Pat. 2,900,306, Slater, issued Aug. 18. 1959; U.S. Pat. 3,255,082, Barton, issued June 7, 1966; U.S. Pat. 4,137,306, Rubino, Issued Jan. 30, 1979; U.S. Pat. 4,226,889, Yuhas, issued Oct. 7, 1980; U.S. Pat. 4,944,937, McCall, issued July 31, 1990. See the following patents for DBS gels: U.S. Patents 4,154,816, Roehl et al, issued May 15, 1979; 4,371,645, Mahaffey, issued Feb. 1, 1983; 4,518,582, Schamper et al, issued May 21, 1985; 4,719,102, Randhawa et al, issued Jan. 12, 1988; 4,722,835, Schamper et al, issued Feb. 2, 1988; and 4,725,430, Schamper et al, issued Feb. 16, 1988; 4,781,917, Luebbe et al, November 1, 1988; 4,816,261, Luebbe et al, March 28, 1989; 4,822,602, Sabetelli, issued April 18, 1989. DBS gel sticks are generally regarded as more stable; see Japanese Application 64-62377, Kao, published March 8, 1989, which discloses that certain monosorbitol acetals, particularly fluroinated DBS have good stability than other antiperspirant stick gelling agents known in the art. U.S. Patent 4,429,140, Murai et al, issued Jan. 31, 1984, and European Patent Application 0286522, Salome et al, published January 123, 1988, disclose methods for making fluorinated DBS. U.S. Patent 5,106,999, Gardlik et al, issued April 21, 1992, discloses methods for preparing DBS compounds, particularly meta-substituted halogenated derivatives. The product of the claimed process are purer and have superior gelling properties in antiperspirant stick compositions.

Gel sticks avoid the use of waxy solidifying agents and, therefore, don't suffer from the aesthetic disadvantages associated with wax sticks such as difficult application and high residue. Antiperspirant gel sticks made using soaps, however, do experience problems associated with interaction between the basic soap solidifying agents and the acidic antiperspirant actives. This interaction can result in poor gel formation, reduced efficacy of the actives, and lower stability over time of any gel formed. The interaction may also cause processing difficulties at high temperatures and extended holding times typically encountered during manufacturing. Even the gel sticks made using DBS instead of a basic soap are subject to instability since the acetal portion of the DBS is relatively unstable. In fact, improving the stability of DBS gels has been the object of many patents; see above DBS patents. In addition to the stability issue, DBS must be dissolved in a polar solvent. Polar solvents can contribute to undesirable stick characteristics such as wet, cold and sticky feel on the skin, shrinkage and containment problems due to high volatility, and skin irritation.

The art contains other clear stick compositions that avoid using conventional gellants such as soap, or DBS, to cure the poor stability created by the interaction of such gellants and conventional antiperspirant actives. European Patent Application (EPO) 0 448 278, published September 29, 1991, to Colgate-Palmolive discloses antiperspirant gel sticks comprising an aqueous aluminum active, complexing agent, and hardening agent. EPO 0 373 499, published, June 20, 1988, to Colgate-Palmolive, discloses clear antiperspirant stick compositions comprising an aluminum salt active, water, insoluble volatile emollients, volatile siloxanes, water soluble emollients, coupling agents, and a solublizer. EPO 0 396 137, published November 7 1990, to Gillette, discloses clear anhydrous antiperspirant compositions comprising an anhydrous vehicle such that no significant dissolution of the active antiperspirant in the vehicle. Furthermore, the refractive indicies of the active antiperspirant component and the vehicle are matched. U.S. Patent 4,948,578, Burger et al, issued August 14, 1990, discloses antiperspirant sticks comprising an antiperspirant active, nonionic surfactant, oil, and water. The surfactants are at levels from about 10-40%, and the active no greater that 20%.

While these references disclose sticks which avoid the above mentioned problems associated with soap and DBS sticks, they do have some negatives. For example, using the disclosed reactive materials and surfactants disclosed above typically reduces the effectiveness of the antiperspirant compositions. Additionally, some of the disclosed compositions require processes steps which involve mixing several viscous phases at specific times, and at specific temperatures. Furthermore, the gels formed from the disclosed polymers and emulsions do not maintain the desirable aesthetics of the typical soap or DBS gel sticks disclosed above.

Gels of non-polar liquids and n-acyl amino acids and derivatives thereof used as gelling agents is disclosed in U.S. Pat. 3,969,087, issued July 13, 1976. This patent discloses gelling non-polar materials such as fuels, motor oils, paints, edible oils, and cosmetics. Cosmetic stick compositions utilizing n-acyl amino acids as gelling agents is disclosed in Japanese Patent Application 1-207223, published August 21, 1989, (hereinafter'223), and Japanese Patent Application 2-180805, published July 13, 1988 (hereinafter '805). In '223, the n-acylamino acid amide is combined with a starch fatty acid ester to form a cosmetic gel compositions. The n-acylamino acid amide must be highly soluble in certain oils which are typically used in the cosmetic arts. In '805, the cosmetic gel compositions disclosed therein have improved transparency. The gel comprises methylphenyl polysiloxanes and n-lauroyl-L-glutamic acid dibutylamide. Gels absent the methylphenyl polysiloxanes have poor transparency as compared with those claimed therein. None of the above Japanese references teach using the presently disclosed gel compositions to overcome the stability problems noted above since the cosmetic gel compositions taught therein do not contain antiperspirant actives.

It is, therefore, the object of the present invention to provide antiperspirants gel sticks having excellent efficacy, and reduced residue on the skin, which are stable. It is also the object of the present invention to provide antiperspirant gel sticks which can be made by conventional techniques known in the art. Lastly, the object of the present invention is to provide antiperspirant sticks having excellent aesthetics and minimal skin irritation.

### SUMMARY OF THE INVENTION

The present invention are antiperspirant gel stick compositions comprising:
a. a gelling agent;
b. a liquid base material; and
c. an antiperspirant active.
wherein the gelling agent is selected from the group consisting of n-acyl amino acid amides, and mixtures of n-acyl amino acid amides and 12-hydroxystearic acid.

These antiperspirant gel sticks have superior stability, low residue on the skin, low skin irritation, and excellent aesthetic characteristics. Furthermore, these compositions can be easily prepared by conventional techniques known in the art.

### DETAILED DESCRIPTION OF THE INVENTION

The present invention comprises the ingredients disclosed below.

### A. Gelling Agents:

The gelling agents of the present invention are n-acyl amino acid amides, or mixtures of n-acyl amino acid amides and 12-hydroxystearic acid. The level of the gelling agent is from about 1% to about 15%, preferably from 3% to about 12%, most preferably from about 5% to about 10% total weight of the gel composition. When the mixture of a n-acyl amino acid amide and 12-hydroxystearic acid is the gelling agent, the ratio of n-acyl amino acid amide to 12-hydroxystearic acid is from 1:20 to about 2:1, preferably from about 1:10 to about 1:1, and most preferably from about 1:5 to about 1:2, wherein the level of n-acyl amino acid amide is at least about 0.1% total weight of the gel composition.

N-acyl amino acid amides are disclosed in U.S. Pat. 3,969,087, Saito et al, issued July 13, 1976; incorporated herein by reference. The preferred n-acyl amino acid amides are prepared from glutamic acid, lysine, glutamine, aspartic acid and mixtures thereof. Particularly preferred are n-acyl glutamic acid amides corresponding to the following formula: 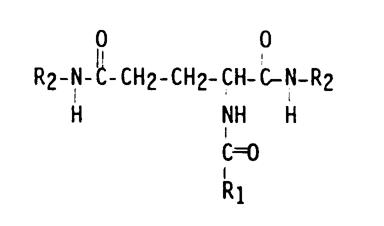
wherein R₁ is an aliphatic hydrocarbon radical having from about 12 to about 22 carbon atoms, and R₂ is an aliphatic hydrocarbon radical having from about 4 to about 12 carbon atoms. Non-limiting examples of these include n-lauroyl-L-glutamic acid dibutyl amide, n-stearoyl-L-glutamic acid diheptyl amide, and mixtures thereof. Most preferred is n-lauroyl-L-glutamic acid dibutyl amide.

Although the presently disclosed antiperspirant gel sticks can be made solely with the n-acyl amino acid amide as the gelling agent, it has been found that incorporating 12-hydroxystearic acid into the gelling agent is advantageous. When 12-hydroxystearic acid is used in combination with the n-acyl amino acid amide, the level of said gellant can be reduced while maintaining desirable stick characteristics such as hardness, low residue, and easy application qualities. Furthermore, the 12-hydroxystearic acid is suprisingly an excellent solvent for the n-acyl amino acid amide. Therefore, the processing temperatures used to make the gel stick can be reduced. Lower processing temperatures reduces the tendency of the antiperspirant active and the perfumes used therein to degrade during processing. Also the lower processing temperatures improves the compatibility of the above components with other gel stick components having lower boiling points, and packaging materials used to contain the stick.

The 12-hydroxystearic acid useful in the present invention is known in the art. Japanese Patent Application 2-180805, cited supra, and Japanese Patent Application 2-264707, published October 31, 1990, disclose using 12-hydroxystearic acid in making cosmetic gel compositions. In fact, 2-180805 discloses cosmetic gel compositions comprising phenylmethylsiloxanes and n-lauroyl-L-glutamic acid dibutyl amide in an amount necessary to achieve transparency and a gel consistency to facilitate easy spreading of the gel over the skin's surface.

### B. Liquid Base Material

The base matrix of gel stick compositions of the present invention is formed by combining the above gelling agent with the liquid base material. As used herein, the term liquid is defined as materials which are liquids at ambient conditions. It is important that the liquid base material selected is of a type, and at such a level to sufficiently solubilize the gelling agent when heated, and form a gel when cooled to ambient temperature. The liquid base material must be compatible with the gelling agent so that the mixture of the two does not phase separate during processing. Furthermore, the liquid base materials are selected in order to provide aesthetic benefits, such as as emolliency, low tack or minimized visible residue, without substantial interference with the effectiveness of the antiperspirant active component. Lastly, the particular liquid base material should be safe for human application.

The liquid base materials of the present invention are preferably used at levels from about 10% to about 95%, more preferably from about 30% to about 80% of the composition. These liquid base materials generally comprise non-polar emollient oils having solubility parameters from about 5 to about 11, or mixtures thereof such that the average solubility parameter of the liquid base material is from about 6 to about 10. Hence, a single non-polar emollient oil comprising the liquid base material may be used which itself has a solubility parameter in the range of from about 6 to about 10. Alternatively, a mixture of non-polar emollients may be used as the liquid base material herein, each having a solubility parameter in the range of from about 5 to 11, such that the average solubility parameter of the mixture is from about 6 to about 10. Solubility parameters and the means to determine them are disclosed by C.D. Vaughan, "Solubility Effects in Product, Package, Penetration and Preservation" 103 Cosmetics and Toiletries 47-69, October, 1988; and C.D. Vaughan, " Using Solubility Parameters in Cosmetics Formulation", 36 J Soc. Cosmetic Chemists 319-333, Sept/Oct, 1985.

Non-polar emollients useful in the present invention are disclosed in Cosmetics, Science, and Technology, Vol. 1, 27-104 edited by Balsam and Sagarin, 1972; U.S. Pat. 4,202,879, Shelton, issued May 13, 1980; and U.S. Pat. 4,816,261, issued March 28, 1989. Non-polar emollient oils particularly useful in the present invention are selected from the group consisting of silicone oils; hydrocarbon oils; fatty alcohols; fatty acids; esters of mono and dibasic carboxylic acids and mono and polyhydric alcohols; polyoxyethylene, polyoxypropylene, mixtures of polyoxyethylene and polyoxypropylene ethers of fatty alcohols; and mixtures thereof. The emollients useful in the present invention may be either saturated or unsaturated, have an aliphatic character and be straight or branched chained or contain alicyclic or aromatic rings.

Silicone oils, as disclosed above, include both volatile and non-volatile silicone oils. Volatile silicone oils are particularly useful as the non-polar emollients in the liquid base material since they endow the gel stick with highly desirable aesthetics. Volatile silicone oils preferably comprise at least about 50%, more preferably about 60%, and most preferably about 70% of the liquid base material.

Volatile silicone oils are disclosed in U.S. Pat. 4,781,917, Luebbe et al, issued Nov. 1, 1988 reference. A description of various volatile silicones materials is found in Todd et al., "Volatile Silicone Fluids for Cosmetics", *Cosmetics and Toiletries*, *91*:27-32 (1976).

As previously disclosed, non-polar emollients having solubility parameters below 6 may be used in the present invention. Therefore, volatile silicone oils having a solubility parameter from about 5 to about 6 may be useful in the present invention. These volatile silicone oils are selected from the group consisting of cyclic volatile silicones corresponding to the formula 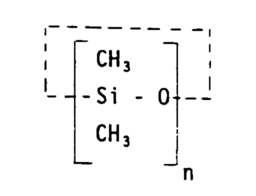
wherein n is from about 3 to about 7; and linear volatile silicones corresponding to the formula

(CH₃)₃Si-O-[Si(CH₃)₂O]ₘ-Si(CH₃)₃

wherein m is from about 1 to about 7. Linear volatile silicones generally have viscosities of less than about 0.05 cm²/s (5 centistokes) at 25°C, whereas the cyclic silicones have viscosities of less than about 0.1 cm²/s (10 centistokes). Examples of volatile silicones useful in the present invention include: Dow Corning 344, Dow Corning 345, and Dow Corning 200 (commercially available from Dow Corning Corp.); GE 7207 and 7158 (commercially available from General Electric Co.); and SWS-03314 (commercially available from SWS Silicones Corp.).

Volatile silicone oils having solubility parameters below 6 are combined with additional non-polar emollient oils having solubility parameters preferably from about 7 to about 11 to bring the average solubility parameter of the liquid base material to from about 6 to about 10. Additional non-polar emollient oils preferred for use in combination with volatile silicone oils having solubility parameters below 6 are selected from the group consisting fatty alcohols having from about 12-22 carbon atoms; fatty acids having from about 12-22 carbon atoms; esters of monobasic carboxylic acids and alcohols having from about 14-30 carbon atoms; esters of dibasic carboxylic acids and alcohols having from about 10-30 carbon atoms; esters of polyhydric alcohols and carboxylic acids having from about 5 to 22 carbon atoms; ethoxylated, propoxylated, and mixtures of ethoxylated and propoxylated ethers of fatty alcohols with from about 12 to 22 carbon atoms and a degree of ethoxylation and propoxylation of below about 50; and mixtures thereof. Preferred are propoxylated ethers of C₁₄-C₁₈ fatty alcohols having a degree of propoxylation below about 50, esters of C₂-C₈ alcohols and C₁₂-C₂₂ carboxylic acids (e.g. ethyl myristate, isopropyl palmitate), esters of C₁₂-C₂₂ alcohols and benzoic acid (e.g. Finsolv TN supplied by Finetex), diesters of C₂-C8 alcohols and adipic, sebacic, and phthalic acids (eg., diisopropyl sebacate, diisopropyl adipate, di-n-butyl phthalate), polyhydric alcohol esters of C₆-C₂₂ carboxylic acids (e.g., propylene glycol dicaprate/dicaprylate, propylene glycol isostearate); and mixtures thereof.

The non-volatile silicone oils useful in the present invention are essentially non-volatile polyalkyl siloxanes selected from the group consisting of polyalkylsiloxanes, polyarylsiloxanes, polyalkylarylsiloxanes, polyethersiloxane copolymers, and mixtures thereof. Examples of these include polydimethyl siloxanes having viscosities of from about 0.05 to about 1000 cm²/s (5 to about 100,000 centistokes) at 25°C. Among the preferred non-volatile silicone emollients useful in the present compositions are the polydimethyl siloxanes having viscosities from about 0.02 to 4 cm²/s (about 2 to about 400 centistokes) at 25°C. Such polyalkyl siloxanes include the Viscasil series (sold by General Electric Company) and the Dow Corning 200 series (sold by Dow Corning Corp.). Polyalkylaryl siloxanes include polymethylphenyl siloxanes having viscosities of from about 15 to about 65 centistokes at 25°C. These are available, for example, as SF 1075 methylphenyl fluid (sold by General Electric Company) and 556 Cosmetic Grade Fluid (sold by Dow Corning Corp.). Useful polyether siloxane copolymers include, for example, a polyoxyalkylene ether copolymer having a viscosity of about 12 to 15 cm²/s (about 1200 to 1500 centistokes) at 25°C. Such a fluid is available as SF1066 organosilicone surfactant (sold by General Electric Company). Polysiloxane ethylene glycol ether copolymers are preferred copolymers for use in the present compositions.

Non-volatile paraffinic hydrocarbon oils or fluids useful in the present invention include mineral oils and certain branched-chain hydrocarbons. Examples of these fluids are disclosed is U.S. 5,019,375, Tanner et al, issued May 28, 1991.

### C. Antiperspirant Active

The compositions of the present invention also contain an astringent antiperspirant active. These actives are used at levels from about about 1% to about 60%, preferably from about 5% to about 35%, of the antiperspirant gel composition. This active may be incorporated either in solubilized or particulate form. These weight percentages are calculated on an anhydrous metal salt basis (exclusive of glycine, the salts of glycine, or other complexing agents). If used in particulate form, the material preferably has a particle size of from about 1 to about 100 microns, preferably from about 1 to about 50 microns, and have a high bulk density (e.g., greater than about 0.7 g/cm³). Such materials include, for example, many aluminum or zirconium astringent salts or complexes and are well known in the antiperspirant art.

Any aluminum astringent antiperspirant salt or aluminum and/or zirconium astringent complex can be employed herein. Salts useful as astringent antiperspirant salts or as components of astringent complexes include aluminum halides, aluminum hydroxyhalides, zirconyl oxyhalides, zirconyl hydroxyhalides, and mixtures of these materials.

Aluminum salts of this type include aluminum chloride and the aluminum hydroxyhalides having the general formula Al₂(OH)ₓQ_{y}.XH₂O where Q is chlorine, bromine or iodine; where x is from about 2 to about 5, and x+y = about 6, and x and y do not need to be integers; and where X is from about 1 to about 6. Aluminum salts of this type can be prepared in the manner described more fully in U.S. Patent 3,887,692, Gilman, issued June 3, 1975, and U.S. Patent 3,904,741, Jones and Rubino, issued September 9, 1975.

The zirconium compounds which are useful in the present invention include both the zirconium oxy salts and zirconium hydroxy salts, also referred to as the zirconyl salts and zirconyl hydroxy salts. These compounds may be represented by the following general empirical formula:

ZrO(OH)_{2-nz}B_{z}

wherein z may vary from about 0.9 to about 2 and need not be an integer, n is the valence of B, 2-nz is greater than or equal to 0, and B may be selected from the group consisting of halides, nitrate, sulfamate, sulfate, and mixtures thereof. Although only zirconium compounds are exemplified in this specification, it will be understood that other Group IVB metal compounds, including hafnium, can be used in the present invention.

As with the basic aluminum compounds, it will be understood that the above formula is greatly simplified and is intended to represent and include compounds having coordinated and/or bound water in various quantities, as well as polymers, mixtures and complexes of the above. As will be seen from the above formula, the zirconium hydroxy salts actually represent a range of compounds having various amounts of the hydroxy group, varying from about 1.1 to only slightly greater than 0 groups per molecule.

Several types of antiperspirant complexes utilizing the above antiperspirant salts are known in the art. For example, U.S. Patent 3,792,068, Luedders et al., issued February 12, 1974, discloses complexes of aluminum, zirconium and amino acids, such as glycine. Complexes such as those disclosed in the Luedders et al. patent and other similar complexes are commonly known as ZAG. ZAG complexes are chemically analyzable for the presence of aluminum, zirconium and chlorine. ZAG complexes useful herein are identified by the specification of both the molar ratio of aluminum to zirconium (hereinafter "Al:Zr" ratio) and the molar ratio of total metal to chlorine (hereinafter "Metal:Cl" ratio). ZAG complexes useful herein have an Al:Zr ratio of from about 1.67 to about 12.5 and a Metal:Cl ratio of from about 0.73 to about 1.93.

Preferred ZAG complexes are formed by
(A) co-dissolving in water
   (1) one part Al₂(OH)₆₋ₘQₘ, wherein Q is an anion selected from the group consisting of chloride, bromide and iodide, and m is a number from about 0.8 to about 2.0;
   (2) x parts ZrO(OH)₂₋ₐQₐ.nH₂O, where Q is chloride, bromide or iodide; where a is from about 1 to about 2; where n is from about 1 to about 8; and where x has a value of from about 0.16 to about 1.2;
   (3) p parts neutral amino acid selected from the group consisting of glycine, dl-tryptophane, dl-β-phenylalanine, dl-valine, dl-methionine and β-alanine, and where p has a value of from about 0.06 to about 0.53;
(B) co-drying the resultant mixture to a fryable solid; and
(C) reducing the resultant dried inorganic-organic antiperspirant complex to particulate form.

A preferred aluminum compound for preparation of such ZAG type complexes is aluminum chlorhydroxide of the empirical formula Al₂(OH)₅Cl.2H₂O. Preferred zirconium compounds for preparation of such ZAG-type complexes are zirconyl hydroxychloride having the empirical formula ZrO(OH)Cl.3H₂O and the zirconyl hydroxyhalides of the empirical formula ZrO(OH)₂₋ₐCl₂.nH₂O wherein a is from about 1.5 to about 1.87, and n is from about 1 to about 7. The preferred amino acid for preparing such ZAG-type complexes is glycine of the formula CH₂(NH₂)COOH. Salts of such amino acids can also be employed in the antiperspirant complexes. See U.S. Patent 4,017,599, Rubino, issued April 12, 1977.

A wide variety of other types of antiperspirant complexes are also known in the art. For example, U.S. Patent 3,903,258, Siegal, issued September 2, 1975, discloses a zirconium aluminum complex prepared by reacting zirconyl chloride with aluminum hydroxide and aluminum chlorhydroxide. U.S. Patent 3,979,510, Rubino, issued September 7, 1976, discloses an antiperspirant complex formed from certain aluminum compounds, certain zirconium compounds, and certain complex aluminum buffers. U.S. Patent 3,981,896, issued September 21, 1976, discloses an antiperspirant complex prepared from an aluminum polyol compound, a zirconium compound and an organic buffer. U.S. Patent 3,970,748, Mecca, issued July 20, 1976, discloses an aluminum chlorhydroxy glycinate complex of the approximate general formula [Al₂(OH)₄Cl][H₂CNH₂-COOH].

Of all the above types of antiperspirant actives, preferred compounds include the 5/6 basic aluminum salts of the empirical formula Al₂(OH)₅Cl.2H₂O; mixtures of AlCl₃.6H₂O and Al₂(OH)₅Cl. 2H₂O with aluminum chloride to aluminum hydroxychloride weight ratios of up to about 0.5; ZAG type complexes wherein the zirconium salt is ZrO(OH)Cl.3H₂O, the aluminum salt is Al₂(OH)₅Cl. 2H₂O or the aforementioned mixtures of AlCl₃.6H₂O and Al₂(OH)₅ Cl.2H₂O wherein the total metal to chloride molar ratio in the complex is less than about 1.25 and the Al:Zr molar ratio is about 3.3, and the amino acid is glycine; and ZAG-type complexes wherein the zirconium salt is ZrO(OH)₂₋ₐClₐ.nH₂O wherein a is from about 1.5 to about 1.87 and n is from about 1 to about 7, the aluminum salt is Al₂(OH)₅Cl.2H₂O, and the amino acid is glycine.

Solubilized antiperspirant actives which may be utilized in the present invention are also well known in the art. These materials utilize monohydric or polyhydric alcohols or water to solubilize the antiperspirant active before it is incorporated into the product. The levels of these polar solvents is less than 25%, and preferably less than 15% of the composition. Examples of such actives are taught, for example, in U.S. Patent 4,137,306, Rubino, issued January 30, 1979, and European Published Application 0295070, published December 14, 1988.

### D. Optional Ingredients:

The compositions of the present invention may also contain optional components which act as additional active or modify the physical characteristics of the composition or the components making up said compositions. Such components are well known in the art. A non-limiting group of these optional components include colorants, perfumes, thickeners, emulsifiers, bacteriostats, fungistats, and mixtures thereof.

Emulsifiers are particularly useful in the present invention. These emulsifiers include non-ionic surfactants useful for forming water-in-oil emulsions. The level of emulsifiers used in the present invention is typically less than about 10%, preferably less than about 5%. Examples of these emulsifiers include polyoxyethylene ethers of fatty alcohols, and polyoxyethylene -polysiloxane copolymers. Such emulsifiers are disclosed by EPO Application 373,424 Raleigh et al.

Thickeners are also useful in the presently invention. Their selection and the level they are used at should be so as not to significantly affect the aesthetics of the gel composition.
Typical levels of thickners are at levels of less than about 5%. Examples of said thickeners are disclosed in U.S. Pat. 4,985,238, Tanner et al., issued Jan. 15, 1991. These thickeners include wax-like materials such as beeswax, cerasin, hydrogenated castor oil, synthetic waxes such as Fisher Tropsch waxes, microcrystalline waxes, polyethylene waxes, and mixtures thereof.

Particulate and filler materials may also be included in the present compositions. These materials are typically used at levels from about 0.5% to about 5%, preferably not more than 3%. Such materials are disclosed in U.S. Pat. 5,019,375, Tanner et al., issued May 28, 1991. Suitable filler materials include collodial silica (such as Cab-O-Sil, sold by Cabot Corp), clays (such as bentonite), hydrophobic (quaternized) clays, silica/alumina thickeners, silicate powders such as talc, alumina silicate, and magnesium silicate, modified corn starches, metallic stearates, and mixtures thereof. The use of such fillers as stabilizing agents in cosmetic sticks is disclosed in U.S. Pat. 4,126,679, Davy et al., issued November 21, 1987. Examples of other particulate materials include particulate hydrophilic polymers such as cellulose ether polymers, modified starches, polyamides, and polypeptides.

### METHODS OF MANUFACTURE

The present invention may be made by using any of the typical methods known to those skilled in the art, and disclosed in Gels and Sticks Formulary, 99 Cosmetics & Toiletries 77-84, 1984. Methods found particularly useful follow below:
Combine the gelling agent and the liquid base material into a vessel equipped with a heat source. Heat the mixture to about 130°C to about 150°C with stirring, until the mixture forms a homogeneous solution. Add the antiperspirant active and other ingredients, such as fragrances and colors, into the above vessel with stirring. Pour the mixture into containers allowing them to cool. Alternatively, the antiperspirant active may be added along with the gelling agent and the liquid base material in the first step.

When using the combination of n-acyl amino acid amide and 12-hydroxystearic acid, the above method may be used, except the temperature of the mixture is lowered to about 80°C to about 130°C.

### METHODS FOR USE

The present invention provides methods for preventing perspiration and malodor associated with human perspiration. These methods comprise applying to the skin of a human a safe and effective amount of the antiperspirant gel of the present invention. The term "a safe and effective amount" as used herein, is an amount which is effective in eliminating or substantially reducing malodor associated with human underarm perspiration while being safe for human use at a reasonable risk/benefit ratio.

### EXAMPLES

The following examples further describe and demonstrate embodiments within the scope of the present invention. These examples are solely for the purpose of illustration and are not to be construed as limitations of the present invention.

The levels of the components in the examples below are expressed by total weight of the composition.

| | EXAMPLES | | | | | | |
|---|---|---|---|---|---|---|---|
| | I | II | III | IV | V | VI | VII |
| N-Lauroyl-L-glutamic acid-di-n-butyl amide¹ | 8 | 4 | 10 | 6 | 8 | 13 | 9 |
| Cyclomethicone D-5² | | 28 | 40 | 49 | 47 | 42 | 50 |
| Polyphenylmethylsiloxane³ | | | | 10 | | | |
| Light mineral oil⁴ | 15 | | | | | 5 | |
| Panalane-L-14E⁵ | | | | | 10 | | |
| Isopropyl Myristate | | 28 | | 10 | | 20 | 13 |
| PPG-3 Myristyl Ether | | | 30 | | | | 3 |
| C12-15 Alcohols Benzoate⁶ | 62 | | | | | | |
| Diisopropyl Sebacate⁷ | | | | | 10 | | |
| Aluminum Zirconium Trichlorhydrex Gly⁸ | 15 | 40 | 20 | 25 | 25 | 20 | |
| Aluminum Chlorohydrate⁹ | | | | | | | 25 |
| | $\overline{\text{100}}$ | $\overline{\text{100}}$ | $\overline{\text{100}}$ | $\overline{\text{100}}$ | $\overline{\text{100}}$ | $\overline{\text{100}}$ | $\overline{\text{100}}$ |

| | | | | | | | |
|---|---|---|---|---|---|---|---|
| 1) GP-1 supplied by Ajinomoto, Inc. | | | | | | | |
| 2) Dow Corning 245 Fluid - cyclic polydimethylsiloxane | | | | | | | |
| 3) Dow Corning 556 Fluid | | | | | | | |
| 4) Benol White Mineral Oil supplied by Witco Chemical Corp. | | | | | | | |
| 5) polyisobutene supplied by Amoco Chemical Company | | | | | | | |
| 6) Finsolv TN supplied by Finetex | | | | | | | |
| 7) Schercemol DIS supplied by Sher Chemicals Inc. | | | | | | | |
| 8) supplied by Westwood Chemical Co. | | | | | | | |
| 9) Westchlor DM200 supplied by Westwood Chemical Co. | | | | | | | |

The antiperspirant gel stick compositions were prepared by combining the N-lauroyl glutamic acid amide and liquid base components. Those components were heated to about 130-150°C to form a clear solution. Once clear the solution is cooled and held at approximately 115-140°C at which time the antiperspirant active is added. The active is mixed thoroughly into the composition and the mixture is poured into containers. Upon cooling a stable antiperspirant gel stick is obtained. Similar results were obtained when using N-stearoyl-L-glutamic acid diheptyl amide for 1) above.

| | VIII | IX | X | XI | XII | XIII | XIV | XV |
|---|---|---|---|---|---|---|---|---|
| N-Lauroyl-L-glutamic acid-di-n-butyl amide ¹ | 4 | 5 | 1 | 3 | 2 | 2 | 2 | 1 |
| 12-hydroxystearic acid | 2 | 5 | 5 | 6 | 7 | 3 | 6 | 12 |
| Cyclomethicone D-5² | | 40 | 49 | 39 | 43 | 40 | 43 | 46 |
| Polyphenylmethylsiloxane³ | | | | 3 | | | 5 | |
| Light mineral oil⁴ | 23 | | | | | | | |
| Panalane-L-14E⁵ | | 15 | 10 | 11 | | | | |
| Isopropyl Myristate | | 15 | 15 | 16 | | | 11 | |
| Isopropyl Alcohol | | | | | 18 | | | |
| Captex 200⁶ | | | | | | 15 | | |
| C12-C15 Alcohols Benzoate⁷ | | | | | | | 8 | |
| PPG-3 Myristyl Ether | | | | | | | | 26 |
| Diisopropyl Sebacate⁸ | 43 | | | | | | | |
| Aluminum Zirconium Trichlorhydrex Gly⁹ | 25 | 20 | 20 | 20 | | 40 | 25 | |
| Aluminum Chlorohydrate¹⁰ | | | | | 30 | | | 10 |
| Talc | 3 | | | 2 | | | | 5 |
| | $\overline{\text{100}}$ | $\overline{\text{100}}$ | $\overline{\text{100}}$ | $\overline{\text{100}}$ | $\overline{\text{100}}$ | $\overline{\text{100}}$ | $\overline{\text{100}}$ | $\overline{\text{100}}$ |

| | | | | | | | | |
|---|---|---|---|---|---|---|---|---|
| 1) GP-1 supplied by Ajinomoto, Inc. | | | | | | | | |
| 2) Dow Corning 245 Fluid-cyclic polydimethylsiloxane | | | | | | | | |
| 3) Dow Corning 556 Fluid | | | | | | | | |
| 4) Benol White Mineral Oil supplied by Witco Chemical Corp. | | | | | | | | |
| 5) polyisobutene supplied by Amoco Chemical Company | | | | | | | | |
| 6) propylene glycol dicaprate/dicaprylate supplied by Capital City Products | | | | | | | | |
| 7) Finsolv TN supplied by Finetex | | | | | | | | |
| 8) Schercemol DIS supplied by Scher Chemicals Inc. | | | | | | | | |
| 9) supplied by Westwood Chemical Co. | | | | | | | | |
| 10) Westchlor DM200 supplied by Westwood Chemical Co. | | | | | | | | |

The antiperspirant gel stick compositions were prepared similarly to Examples I-VII described above except the processing temperatures are decreased by the addition of the 12-hydroxystearic acid. In the present examples, the antiperspirant gel stick compositions were prepared by combining the N-lauroyl-L-glutamic acid amide, 12-hydroxystearic acid and liquid base components. These components were heated to approximately 80-130°C to form a clear solution. Once clear, the solution is cooled and held at approximately 65-120°C at which time the antiperspirant active (and other optional components such as filler powders or perfumes) is added. The active is mixed thoroughly into the composition and the mixture is poured into containers. Upon cooling a stable antiperspirant gel stick is obtained. Similar results were obtained when using N-stearoyl-L-glutamic acid diheptyl amide for 1) above.

## Claims

1. An antiperspirant gel stick composition comprising:
a. from 1% to 15%, preferablly from 3% to 12%, and most preferably 5% to 10% of a gelling agent selected from the group consisting of n-acyl amino acid amides, or mixtures of n-acyl amino acid amides and 12-hydroxystearic acid in a ratio of n-acyl amino acid amide to 12-hydroxystearic acid from 1:20 to 2:1, preferably 1:10 to 1:1, and most preferably from 1:5 to 1:2, wherein the the level of n-acyl amino acid amide is at least 0.1% total weight of the composition;
b. from 10% to 95%, preferably 30% to 80% of a liquid base material comprising non-polar emollient oils having a solubility parameter from 5 to 11, and mixtures thereof such that the average solubility parameter of the liquid base material is from 6 to 10; and wherein liquid base material comprises non-polar emollient oils selected from the group consisting of silicone oils; hydrocarbon oils; fatty alcohols; fatty acids; esters of mono and dibasic carboxylic acids and mono and polyhydric alcohols; polyoxyethylene, polyoxypropylene, mixtures of polyoxyethylene and polyoxypropylene ethers of fatty alcohols, wherein the liquid base material contains at least 50%, preferably 60% and most preferably 70% volatile silicone oil.
c. from 1% to 60%, preferably from 5% to 35% of an antiperspirant active comprising inorganic and organic salts of aluminum, zirconium and zinc, and mixtures thereof, preferably antiperspirant active in particulate form and selected from the group consisting of ZAG complexes and actives having the formula
Al₂(OH)ₓQ_{y}.XH₂O
where Q is chlorine, bromine or iodine; where x is from 2 to 5, and x+y = 6, and x and y do not need to be integers; and where X is from 1 to 6, and mixtures thereof.

2. An antiperspirant gel stick composition according to Claim 1 wherein the n-acyl amino acid amide is a n-acyl glutamic acid amide corresponding to the formula 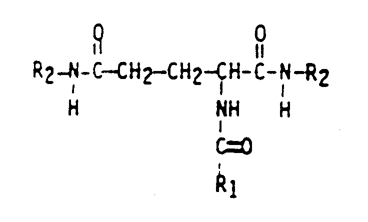 wherein R₁ is an aliphatic hydrocarbon radical having from 12 to 22 carbon atoms, and R₂ is an alphatic hydrocarbon radical having from 4 to 12 carbon atoms.

3. An antiperspirant gel stick composition according to Claims 1 and 2 wherein the n-acyl amino amide is selected from the group consisting of n-lauroyl-L-glutamic acid dibutyl amide, n-stearoyl- L-glutamic acid diheptyl amide, and mixtures thereof.

4. An antiperspirant gel stick composition according to Claims 1 through 3 wherein the liquid base material comprises:
a. volatile silicone oils having a solubility parameter of less than 6, selected from the group consisting of cyclic and linear polydimethyl siloxanes corresponding to the formulas
I. 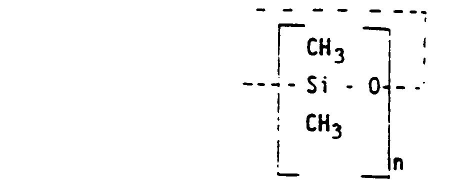
II. (CH₃)₃Si-O-[Si(CH₃)₂O]ₘ-Si(CH₃)₃
wherein n is from 3 to 7, and m is from 1 to 7; and
b. an additional non-polar emollient oil having a solubility parameter from 7 to 11 selected from the group consisting of fatty alcohols having from 12-22 carbon atoms, fatty acids having from 12-22 carbon atoms, esters of monobasic carboxylic acids and alcohols having from 14-30 carbon atoms; esters of dibasic carboxylic acids and alcohols having from 10-30 carbon atoms; esters of polyhydric alcohols and carboxylic acids having from 5 to 22 carbon atoms; ethoxylated, propoxylated, or mixtures of ethoxylated and propoxylated ethers of fatty alcohols with from 12 to 22 carbon atoms, and a degree of ethoxylation and propoxylation below 50; and mixtures thereof;
wherein the average solubility parameter of the liquid base material is from 6 to 10.

5. An antiperspirant gel stick composition according to Claims 1 through 4 wherein the additional non-polar emollient oil is selected from the group consisting of propoxylated ethers of C₁₄-C₁₈ fatty alcohols having a degree of propoxylation below 50, esters of C₂-C₈ alcohols and C₁₂-C₂₂ carboxylic acid, esters of C₁₂-C₂₂ alcohols and benzoic acid, diesters of C₂-C8 alcohols and adipic, sebacic, and phthalic acids, polyhydric alcohol esters of C₆-C₂₂ carboxylic acids, and mixtures thereof.

6. An antiperspirant gel stick composition according to Claims 1 through 5 additionally comprising colorants, perfumes, thickeners, emulsifiers, bacteriostats, fungistats, and mixtures thereof.

7. A process for making antiperspirant gel stick compositions according to Claims 1 through 6 comprising the following steps:
a. combine the gelling agent and liquid base material in vessel equiped with a heat source;
b. stir while heating the mixture to between 130°C and 150°C, preferably 80°C and 120°C obtaining a homogeneous solution;
c. add the antiperspirant active to the heated mixture;
d. stir until uniform;
e. pour the mixture into containers; and
f. cool the mixture until a solid gel is formed.

8. A method of preventing formation of perspiration and malodor by applying to the underarm skin a safe and effective amount of the composition according to Claims 1 through 7.

## Patentansprüche

1. Schweißhemmende Gelstiftzusammensetzung, umfassend:
a. 1% bis 15%, vorzugsweise 3% bis 12%, und am stärksten bevorzugt 5% bis 10% von einem Geliermittel, welches von der Gruppe ausgewählt ist, die aus n-Acylaminosäureamiden oder Gemischen von n-Acylaminosäureamiden und 12-Hydroxystearinsäure in einem Verhältnis von n-Acylaminosäureamid zu 12-Hydroxystearinsäure von 1:20 bis 2:1, vorzugsweise von 1:10 bis 1:1, und am stärksten bevorzugt von 1:5 bis 1:2 besteht, wobei die Menge an n-Acylaminosäureamid mindestens 0,1%, bezogen auf das Gesamtgewicht der Zusammensetzung, beträgt;
b. 10% bis 95%, vorzugsweise 30% bis 80% von einem flüssigen Basismaterial, welches apolare Emolliensöle mit einem Löslichkeitsparameter von 5 bis 11 und Gemische hievon, so daß der durchschnittliche Löslichkeitsparameter des flüssigen Basismaterials von 6 bis 10 beträgt, umfaßt; und worin das flüssige Basismaterial apolare Emolliensöle umfaßt, welche von der Gruppe ausgewählt sind, die aus Siliconölen; Kohlenwasserstoffölen; Fettalkoholen; Fettsäuren; Estern von ein- und zweibasigen Carbonsäuren und ein- und mehrwertigen Alkoholen; Polyoxyethylen, Polyoxypropylen, Gemischen von Polyoxyethylen- und Polyoxypropylenethern von Fettalkoholen besteht, wobei das flüssige Basismaterial mindestens 50%, vorzugsweise 60% und am stärksten bevorzugt 70%, flüchtiges Siliconöl enthält;
c. 1% bis 60%, vorzugsweise 5% bis 35% eines schweißhemmend wirksamen Materials, umfassend anorganische und organische Salze von Aluminium, Zirconium und Zink, und Gemische hievon, vorzugsweise ein schweißhemmend wirksames Mittel in Teilchenform, welches von der Gruppe ausgewählt ist, die aus ZAG-Komplexen und wirksamen Mitteln der Formel
Al₂(OH)ₓQ_{y}.XH₂O,
worin Q für Chlor, Brom oder Iod steht; x von 2 bis 5 beträgt; und x+y 6 ist; und x und y keine ganzen Zahlen sein müssen; und worin X von 1 bis 6 ist, und Gemischen hievon besteht.

2. Schweißhemmende Gelstiftzusammensetzung nach Anspruch 1, worin das n-Acylaminosäureamid ein n-Acylglutaminsäureamid der Formel 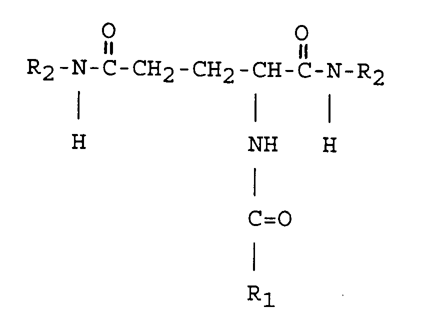 ist, worin R₁ einen aliphatischen Kohlenwasserstoffrest mit 12 bis 22 Kohlenstoffatomen bedeutet und R₂ ein aliphatischer Kohlenwasserstoffrest mit 4 bis 12 Kohlenstoffatomen ist.

3. Schweißhemmende Gelstiftzusammensetzung nach den Ansprüchen 1 und 2, worin das n-Acylaminosäureamid von der aus n-Lauroyl-L-glutaminsäuredibutylamid, n-Stearoyl-L-glutaminsäurediheptylamid und Gemischen hievon bestehenden Gruppe ausgewählt ist.

4. Schweißhemmende Gelstiftzusammensetzung nach den Ansprüchen 1 bis 3, worin das flüssige Basismaterial
a. flüchtige Siliconöle mit einem Löslichkeitsparameter von weniger als 6, welche von der Gruppe ausgewählt sind, die aus cyclischen und linearen Polydimethylsiloxanen der Formeln
I. 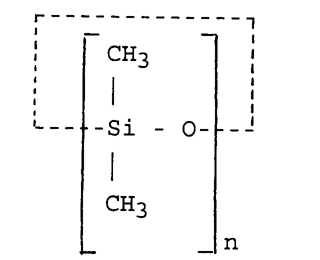
II. (CH₃)₃-Si-O-[Si(CH₃)₂O]ₘ-Si(CH₃)₃
besteht, worin n von 3 bis 7 beträgt und m von 1 bis 7 ist; und
b. ein zusätzliches apolares Emolliensöl mit einem Löslichkeitsparameter von 7 bis 11 umfaßt, welches von der Gruppe ausgewählt ist, die aus Fettalkoholen mit 12 bis 22 Kohlenstoffatomen, Fettsäuren mit 12 bis 22 Kohlenstoffatomen, Estern von einbasigen Carbonsäuren und Alkoholen mit 14 bis 30 Kohlenstoffatomen; Estern von zweibasigen Carbonsäuren und Alkoholen mit 10 bis 30 Kohlenstoffatomen; Estern von mehrwertigen Alkoholen und Carbonsäuren mit 5 bis 22 Kohlenstoffatomen; ethoxylierten, propoxylierten oder Gemischen von ethoxylierten und propoxylierten Ethern von Fettalkoholen mit 12 bis 22 Kohlenstoffatomen, und einem Ethoxylierungs- und Propoxylierungsgrad unter 50; und Gemischen hievon besteht;
wobei der durchschnittliche Löslichkeitsparameter des flüssigen Basismaterials von 6 bis 10 beträgt.

5. Schweißhemmende Gelstiftzusammensetzung nach den Ansprüchen 1 bis 4, wobei das zusätzliche apolare Emolliensöl von der Gruppe ausgewählt ist, welche aus propoxylierten Ethern von C₁₄-C₁₈-Fettalkoholen mit einem Propoxylierungsgrad unter 50, Estern von C₂-C₈-Alkoholen und C₁₂-C₂₂-Carbonsäuren, Estern von C₁₂-C₂₂-Alkoholen und Benzoesäure, Diestern von C₂-C₈-Alkoholen und Adipinsäure, Sebacinsäure und Phthalsäure, Estern von mehrwertigen Alkoholen und C₆-C₂₂-Carbonsäuren und Gemischen hievon besteht.

6. Schweißhemmende Gelstiftzusammensetzung nach den Ansprüchen 1 bis 5, welche zusätzlich Färbemittel, Parfums, Verdickungsmittel, Emulgatoren, bakteriostatische Mittel, fungistatische Mittel und Gemische hievon umfaßt.

7. Verfahren zur Herstellung schweißhemmender Gelstiftzusammensetzungen nach den Ansprüchen 1 bis 6, welches die folgenden Schritte umfaßt:
a. Vereinigen des Geliermittels und des flüssigen Basismaterials in einem mit einer Wärmequelle ausgerüsteten Gefäß;
b. Rühren während Erhitzen des Gemisches auf 130°C bis 150°C, vorzugsweise auf 80°C bis 120°C, wobei eine homogene Lösung erhalten wird;
c. Zugeben des schweißhemmend wirksamen Mittels zum erhitzten Gemisch;
d. Rühren bis zur Einheitlichkeit;
e. Leeren des Gemisches in Behälter; und
f. Abkühlen des Gemisches bis zur Ausbildung eines festen Geles.

8. Verfahren zur Verhinderung von Schweißbildung und schlechtem Geruch durch Aufbringen einer sicheren und wirksamen Menge der Zusammensetzung nach den Ansprüchen 1 bis 7 auf die Haut des Unterarms.

## Revendications

1. Composition de stick de gel antitranspiration comprenant:
a. de 1% à 15%, de préférence de 3% à 12%, et plus particulièrement de 5% à 10%, d'un agent gélifiant choisi dans le groupe constitué par les amides d'acide aminé n-acylé, ou les mélanges d'amides d'acide aminé n-acylé et d'acide 12-hydroxystéarique dans un rapport de l'amide d'acide aminé n-acylé à l'acide 12-hydroxystéarique de 1:20 à 2:1, de préférence de 1:10 à 1:1, et plus particulièrement de 1:5 à 1:2, dans lequel la proportion d'amide d'acide aminé n-acylé représente au moins 0,1% du poids total de la composition;
b. de 10% à 95%, de préférence de 30% à 80% d'une substance de base liquide comprenant des huiles émollientes non polaires ayant un paramètre de solubilité de 5 à 11, et leurs mélanges, de telle sorte que le paramètre de solubilité moyen de la substance de base liquide soit de 6 à 10; et dans laquelle la substance de base liquide comprend des huiles émollientes non polaires choisies dans le groupe constitué par les huiles de silicone; les huiles hydrocarbonées; les alcools gras; les acides gras; les esters d'acides mono- et dicarboxyliques et de monoalcools et polyols; les éthers de polyoxyéthylène, de polyoxypropylène, de mélanges de polyoxyéthylène et de polyoxypropylène d'alcools gras, dans laquelle la substance de base liquide contient au moins 50%, de préférence 60% et plus particulièrement 70% d'huile de silicone volatile ;
c. de 1% à 60%, de préférence de 5% à 35%, d'une substance active antitranspiration comprenant des sels minéraux et organiques d'aluminium, de zirconium et de zinc, et leurs mélanges, de préférence une substance active antitranspiration sous forme particulaire et choisie dans le groupe constitué par les complexes ZAG et les substances actives ayant pour formule
Al₂(OH)ₓQ_{y}·XH₂O
où Q représente un atome de chlore, de brome ou d'iode; où x vaut de 2 à 5, et $\text{x+y = 6}$, et x et y ne sont pas nécessairement des nombres entiers; et où X vaut de 1 à 6, et leurs mélanges.

2. Composition de stick de gel antitranspiration selon la revendication 1, dans laquelle l'amide d'acide aminé n-acylé est un amide d'acide glutamique n-acylé correspondant à la formule 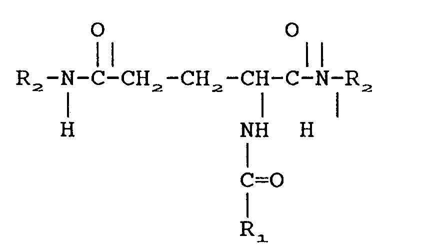 dans laquelle R₁ est radical hydrocarboné aliphatique ayant de 12 à 22 atomes de carbone, et R₂ est un radical hydrocarboné aliphatique ayant de 4 à 12 atomes de carbone.

3. Composition de stick de gel antitranspiration selon les revendications 1 et 2, dans laquelle l'amide d'acide aminé n-acylé est choisi dans le groupe constitué par le dibutylamide d'acide n-lauroyl-L-glutamique, le diheptylamide d'acide n-stéaroyl-L-glutamique, et leurs mélanges.

4. Composition de stick de gel antitranspiration selon les revendications 1 à 3, dans laquelle la substance de base liquide comprend:
a. des huiles de silicone volatiles ayant un paramètre de solubilité inférieur à 6, choisies dans le groupe constitué par les polydiméthylsiloxanes cycliques et linéaires correspondant aux formules
I. 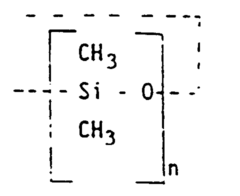
II. (CH₃)₃Si-O-[Si(CH₃)₂O]ₘ-Si(CH₃)₃
dans lesquelles n vaut de 3 à 7, et m vaut de 1 à 7; et
b. une huile émolliente non polaire additionnelle ayant un paramètre de solubilité de 7 à 11 choisie dans le groupe constitué par les alcools gras ayant de 12 à 22 atomes de carbone, les acides gras ayant de 12 à 22 atomes de carbone, les esters d'acides monocarboxyliques et d'alcools ayant de 14 à 30 atomes de carbone; les esters d'acides dicarboxyliques et d'alcools ayant de 10 à 30 atomes de carbone; les esters de polyols et d'acides carboxyliques ayant de 5 à 22 atomes de carbone; les éthers éthoxylés, propoxylés, ou les mélanges d'éthers éthoxylés et propoxylés d'alcools gras ayant de 12 à 22 atomes de carbone, et un degré d'éthoxylation et de propoxylation inférieur à 50; et leurs mélanges;
dans laquelle le paramètre de solubilité moyen de la substance de base liquide est de 6 à 10.

5. Composition de stick de gel antitranspiration selon les revendications 1 à 4, dans laquelle l'huile émolliente non polaire additionnelle est choisie dans le groupe constitué par les éthers propoxylés d'alcools gras en C₁₄-C₁₈ ayant un degré de propoxylation inférieur à 50, les esters d'alcools en C₂-C₈ et d'acide carboxylique en C₁₂-C₂₂, les esters d'alcools en C₁₂-C₂₂ et d'acide benzoïque, les diesters d'alcools en C₂-C₈ et des acides adipique, sébacique et phtalique, les esters de polyol et d'acides carboxyliques en C₆-C₂₂, et leurs mélanges.

6. Composition de stick de gel antitranspiration selon les revendications 1 à 5, qui comprend en outre des colorants, des parfums, des épaississants, des émulsifiants, des bactériostatiques, des fongistatiques, et leurs mélanges.

7. Procédé de préparation des compositions de stick de gel antitranspiration selon les revendications 1 à 6 comprenant les étapes suivantes:
a. combiner l'agent gélifiant et la substance de base liquide dans un récipient équipé d'une source de chaleur;
b. mélanger tout en chauffant le mélange jusqu'à une température comprise entre 130°C et 150°C, de préférence entre 80°C et 120°C, pour obtenir une solution homogène;
c. ajouter la substance active antitranspiration au mélange chauffé;
d. agiter jusqu'à l'obtention d'un mélange uniforme;
e. verser le mélange dans des récipients; et
f. refroidir le mélange jusqu'à la formation d'un gel solide.

8. Procédé pour empêcher la formation de transpiration et de mauvaises odeurs en appliquant sur la peau des aisselles une quantité sans danger et efficace de la composition selon les revendications 1 à 7.
